# EUROPEAN PATENT APPLICATION

(11) **EP 1 402 902 A1**
(43) Date of publication of application: **31.03.2004**
(21) Application number: 02730900.4
(22) Date of filing: 04.06.2002
(51) Int. Cl.: A61K 48/00, A61K 38/00, A61K 45/00, A61P 43/00

(54) **METHOD FOR TREATMENT OF VASCULAR REGENERATION**

(30) Priority: 08.06.2001 JP 2001174919
(71) Applicant: Dnavec Research Inc., Tsukuba-shi, Ibaraki 305-0856 (JP)
(72) Inventor: HAMADA, Hirofumi, Sapporo-shi, Hokkaido 064-0959 (JP); ITO, Yoshinori, Sapporo-shi, Hokkaido 064-0921 (JP); YAMAUCHI, Akihiko, Chuo-ku, Sapporo-shi, Hokkaido 064-0804 (JP); MORIKAWA, Masayuki, Sapporo-shi, Hokkaido 063-0001 (JP)
(74) Representative: Warcoin, Jacques
(86) International application number: PCT/JP2002/005485
(87) International publication number: WO 2002/100441

(57) **Abstract**

The present inventors extensively researched a method of angiogenesis with low vascular permeability by intramuscular injection of angiopoietin-1 prior to VEGF165 into rabbit ischemic hind limb models using a relatively safe plasmid vector. As a result, the present inventors developed a local administration method that produces a high angiogenic induction effect and prevents the adverse effects of edema caused by administration of VEGF alone.

## Description

### Technical Field

The present invention relates to gene therapy that induces normal angiogenesis by expressing or administering angiopoietin prior to the expression or the administration of angiogenic genes.

### Background Art

Recent research for treatment of ischemic diseases has been performed using growth factors that induce angiogenesis. For example, therapeutic effects of fibroblast growth factor 2 (EGF2) (Baffour, R. et al., J. Vasc. Surg. 16 (2): 181-91, 1992) and endothelial cell growth factor (ECGF) (Pu, L. Q. et al., J. Surg, Res. 54 (6): 575-83, 1993) on patients with cardiac infarction and acute limb ischemia have been examined. A recent study has revealed that vascular endothelial growth factor (VEGF)/vascular permeability factor (VPF) promotes vasculogenesis in animal models with myocardial ischemia and limb ischemia (Takeshita, S. et al., Circulation 90 (5 Pt 2): II228-34, 1994; and Takeshita, S. et al., J. Clin, Invest. 93 (2): 662-70, 1994).

Vascular endothelial growth factor (VEGF) was reported as a vascular endothelial cell specific growth factor or as vascular permeability factor (VPF) in 1989, and currently is classified into VEGF A, B, C, D, and E. VEGF A is further divided into 6 different subtypes, and among the subtypes, soluble VEGF 121 and 165 show especially strong vascular growth activity and are now used clinically. It is reported that their effects extend to vasculogenesis during the embryonic period and are enhanced under a low oxygen environment. Moreover, they are involved in NO synthesis and the migration of vascular endothelial cells and vascular endothelial precursor cells. On the other hand, VEGF overexpression may break a balance of angiogenic signals and form "angioma-like" fragile capillary vessels (Carmeliet, P., Nature Med. 6, 1102-1103 (2000)). VEGF gene transfer to vessel walls in vivo may cause significant neointimal formation via angioma-like endothelial proliferation and induce extravasation of red blood cells (Yonemitsu, Y., et al., Lab. Invest. 75, 313-323(1996)). Similar pathological findings were demonstrated in retrovirus-mediated constitutive overexpression of VEGF in myocardium (Lee, R. J., et al., Circulation 102, 898-901 (2000)).

Acute critical limb ischemia, which results from acute obstruction of the major arteries, is caused mainly by thrombotic obstruction and is an ischemic disease important as a target of angiogenic therapy. Delayed treatment of acute critical limb ischemia is quite unsuccessful, often resulting in limb amputation. Moreover, patients with limb amputation have such poor prognosis that one-year survival rates after surgery are only 50%. To solve this problem, techniques for regenerating blood vessels from embryonic stem cells (VEGF receptor Flk1-positive cells) have been investigated (Yamashita, J et al., Nature 408 (2): 92-96, 2000). Blood vessels are composed of endothelial cells and mural cells (pericytes and vascular smooth muscle cells), and a dynamic balance between angiogenesis and blood vessel regression is maintained *in vivo*. It remains unknown what molecular mechanisms control the complex changes such as sprouting, branching, fusion, and intussusception, and how endothelial cells timely achieve functions such as migration, detachment, and adhesion (Suda Toshio, Jikkenigaku (Experimental medicine), May issue, 19 (7): 826-829 (2001)).

Moreover, clinical trials for human gene therapy using angiogenic growth factors have been undertaken, and studies have proceeded to clinically apply this therapy to angiogenesis for treatment of critical limb ischemia. Vascular endothelial growth factor/vascular permeability factor (VEGF/VPF), which are endothelial cell-specific growth factors, are considered as potent therapeutic genes for this purpose. Peripheral vascular diseases, representatives of which include arteriosclerosis obstruction and Burger's disease, show clinical symptoms such as intermittent claudication, resting pain, and tissue damage in the hind limbs (necrosis). Effective treatments are not currently available for patients with resting pain and ischemic ulcer caused by peripheral vascular obstruction. Hind limb amputation is not avoidable in cases where vascular dilation and recovery of blood circulation by surgery cannot be performed. Under these circumstances, a new therapy comprising the formation of collateral circulation by angiogenic factors-therapeutic angiogenesis has been proposed.

Isner et al. have reported angiogenesis using VEGF gene and have demonstrated relatively promising results by means of plasmid-based gene transfer in human (Baumgartner, I., et al., Circulation 97, 1114-1123 (1998); Isner, J. M., et al., J. Vasc. Surg. 28, 964-973 (1998)). Isner et al. began two deferent gene therapy clinical trials: 1) VEGF gene transfer into vessel walls by catheter; and 2) VEGF gene transfer into muscles by injection. Especially, it has been reported that gene transfer using injection showed limb salvage rates of 70 to 80%. Moreover, neither intramuscular nor intravascular gene transfer has shown any toxicity due to immune reaction in clinical trials. More than 100 patients have undergone the treatment and have shown promising results. Related adverse effects and toxicity levels of intramuscular gene transfer of VEGF have been hardly reported at present. However, recent reports indicate that transgenic- (Thurston, G., et al., Science 286, 2511-2514 (1999)) or adenovirus- (Thurston, G., et al., Nature Med. 6, 460-463 (2000)) mediated overexpression of VEGF results in abnormal vasculogenesis in transgene-introduced animals, and that plasmid-based intramuscular VEGF gene transfer showed transient edema in human subjects with ischemic limbs (Baumgartner, I., et al., Circulation 97, 1114-1123 (1998); Isner, J. M., et al., J. Vasc. Surg. 28, 964-973 (1998) Baumgartner, I., et al., Ann Intern Med.; 132, 880-884, (2000)).

No definitive and practical methods are known for ameliorating increased vascular permeability, a drawback in angiogenic therapy by application of VEGF alone.

### Disclosure of the Invention

In order to prevent increased vascular permeability, a drawback of angiogenic therapy using administration of VEGF alone in gene therapies or drug treatments, the present invention provides a method of administering angiopoietin prior to VEGF administration, thereby providing results in highly effective angiogenic induction, without causing the adverse side effects of edema.

Angiopoietin 1 (Ang-1), angiopoietin 2 (Ang-2) and its receptor, Tie-2, are known as regulating factors which specifically interact with vascular endothelial cells, like the VEGF family. Angiopoietins are ligands for the Tie-2 tyrosine kinase receptor, and considered involved in vascular maturation during angiogenesis. Four subtypes are known at present, and subtypes 1 and 2 bind to Tie-2 receptor. Ang-2 is estimated to be an endogenous antagonist of Ang-1 (Saito, T. N; Nature, Vol. 376, 70-74, (1995)). Ang-2 is also known to be induced selectively depending on VEGF function (Oh, H., et al., J. Biol. Chem., 274, 15732-15739 (1999)). The differences between Ang-1 and VEGF are that Ang-1 cannot proliferate vascular endothelial cells, is involved in pericyte junctions, and, unlike VEGF, does not promote vascular permeability. It is known that interaction between endothelial cells and pericytes is important regulatory mechanism in angiogenesis. Pericytes adhere to endothelial cells to thereby function to differentiate endothelial cells and inhibit their proliferation. This mechanism considered to involve TGF-β activation and formation of physical barrier that surround the cells (D'Amore, et al., Proc. Natl. Acad. Sci. USA, 86: 4544-4548 (1989)).

The present inventors studied effects of angiopoietins and reported that pAng-1 improved AG score, blood flow, blood vessel diameter and capillary density when plasmid pAng-1 or pAng-2 was administered in rabbit models (Kou-Gi Shyu, et al., Circulation. 98, 2081-2087 (1998)). Based on this report, the present inventors hypothesized by themselves efficacy for neovascularization by pre-induction of an interaction between pericytes prior to the action of VEGF and conducted experiments to demonstrate the efficacy.

On the other hand, Thurston et al. showed that adenovirus-mediated gene transfer of angiopoietin 1 suppressed the increased permeability induced by VEGF using vascular permeability evaluation models with Evan Blue (Gavin Thurston, Nature Med, Vol. 6, No. 4, 460-463, (2000)). However, another report indicated that co-administration of pAng-1 and pVEGF plasmids to rabbit models showed no difference from administration of pVEGF plasmid alone (Chase, J. K., Arterio. Thromb. Vasc. Bio. 20, 2573-2578 (2000)).

The present inventors focused on the expression timing of both genes, and developed a local administration schedule of pre-administration of angiopoietin prior,to VEGF, which does not cause the adverse side effects of edema and remarkably induces angiogenesis.

The present invention relates to a method of administering angiopoietin prior to VEGF to provide a high angiogenic induction effect without causing the adverse side effects of edema. More specifically, the present invention relates to:
(1) a method of treatment by local administration, said method comprising administering an angiopoietin or a vector encoding an angiopoietin prior to administration of a vector encoding an angiogenic gene or a protein encoded by an angiogenic gene;
(2) the method of (1), wherein said local administration is intramuscular administration;
(3) the method of (1) or (2), wherein said angiogenic gene is VEGF121 or VEGF165; and
(4) the method of any one of (1) to (3), wherein the angiopoietin is angiopoietin 1.

The term "angiogenic gene" used herein indicates a gene that directly or indirectly associated with development, migration, proliferation, and maturation of cells involved in vascular formation.

The present inventors researched extensively for a method of angiogenesis with low vascular permeability, which method comprises injecting Angiopoietin-1 intramuscularly into rabbit ischemic hind limb models, followed by VEGF 165 injection, using relatively safe plasmid vectors, and revealed its therapeutic effect. Target diseases include: 1) peripheral vascular diseases, arteriosclerosis obstruction, and such; 2) heart disorders, angina pectoris, myocardial infarction, and such; 3) nephrosis, nephritis, and such; and 4) pulmonary disorders, nerve disorders, and such, but there is no limitation to the diseases as long as angiogenic induction is effective for the diseases. Treatment targets do not have to be limited to ischemic tissues, but can be tissues that cannot be called ischemic tissues in the strict sense. Such tissues may be affected by diseases and symptoms that can be effectively treated by generation and reconstruction of functional blood vessels. For example, treatment targets include nephritis, diabetic nephrosis; obstinate ulcers; chronic or acute obstinate infectious diseases such as osteomyelitis; and moya-moya disease and other vascular diseases in the neurosurgery field. Any muscle including skeletal, smooth, and cardiac muscle can be used as administration sites. Administration sites are not limited to muscle, but include skin (epidermis and corium), arteries, veins (including the portal vein), lymph duct, kidney, chorion, periosteum, connective tissues, and bone marrow. Methods to substantially obtain therapeutic effects by local administration include direct administration of vectors or proteins, or administration using carriers and vehicles. Vehicles need to be physiologically acceptable and include organics such as biopolymers, inorganics such as hydroxyapatite, specifically, collagen matrix, polylactate polymer or its copolymer, polyethylene glycol polymer or its copolymer, and their chemical derivatives. Moreover, vehicles may be mixtures of these physiologically acceptable materials. Injection tools include industrial products such as conventional medical syringes or continuous injectors that can be kept inside or outside of the body. There is no limitation to vectors as long as the vectors are physiologically acceptable. Any vectors can be used, including adenovirus vectors, adenovirus-associated vectors, retrovirus vectors, lentivirus vectors, herpes simplex virus vectors, vacciniavirus vectors, Sendai virus vectors, and non-viral vector can be used. Vectors can be used in the form of patient-derived cells treated by the vectors.

### Brief Description of the Drawings

Fig. 1 shows the structure of plasmid pCAcc.
Fig. 2 shows the structure of plasmid pCAhVEGF.
Fig. 3 shows the structure of plasmid pCAhAng1.
Fig. 4 shows a protocol for gene transfer into rabbit hind limb ischemic models.
Fig. 5 is a set of photographs showing rabbit scrotum on the ischemic side. These photographs demonstrate that in Group V, scrotum edema and vain dilatation occurred at Day 15 post-gene administration, but pre-administration of Ang-1 prevented scrotum edema induced by VEGF.
Fig. 6 presents selective internal iliac angiographs at Day 40 of six administration schedule groups: (A), Control; (B), Group V; (C) , Group A; (D) , Group A+V, (E) , Group V-A; and (F), Group A-V.
Fig. 7 is a set of representative photomicrographs showing numerous capillary ECs revealed by alkaline phosphatase stain in ischemic thigh muscle. (A), Control; (B), Group V; (C), Group A; (D), Group A+V; (E), Group V-A; and (F), Group A-V; Bar = 50 µm. ECs = endothelial cells.
Fig. 8 is a set of representative photomicrographs showing arterioles covered with smooth muscle cells revealed by smooth muscle cell α-actin stain in ischemic thigh muscle. (A), Control; (B) , Group V; (C) , Group A; (D) , Group A+V; (E), Group V-A; and (F), Group A-V; Bar = 50 µm.

### Best Mode for Carrying out the Invention

Herein below, the present invention will be described in detail using examples; however, it is not to be construed as being limited thereto.

Furthermore, the experiments in the examples were performed according to the following references.
1. Satoshi Takeshita, J. Clin. Invest., 1994, 93: 662-670
2. Yukio Tsurumi, Circulation., 1996, 94: 3281-3290 (Established rabbit hind limb model; demonstrated improvement of AG score, BPR, and capillary density by administration of pVEGF in the rabbit hind limb model; and assessed the above mentioned indexes plus improvement in blood flow.)
3. Iris Baumgartner, Ann. Intern. Med., 2000, 132: 880-884 (Actual assessment of edema in hind limbs of 90 patients who received pVEGF.)
4. Kou-Gi Shyu, Circulation., 1998, 98: 2081-2087 (Assessment of improvements of AG score, blood flow, vessel diameter, and capillary density by administration of pAng1, when pAng1 and pAng2 were administered to rabbit models.)
5. Gavin Thurston, Nature Med, 2000, 6 (4) : 460-463.
6. G. Thurston, Science, 1999, 286: 2511-2514 (Assessment of improvement in suppressed VEGF-induced vascular permeability in vascular permeability evaluation models that received adenovirus vector-mediated Ang1 transfer)
7. Lioubov Poliakova, J. Thorac. Cardiovasc. Surg., 1999, 118: 339-347. Time course assessment of changes in scrotum size and hind limb circumference in rabbit models which received adenovirus vector-mediated VEGF transfer )

Data are shown as mean ± SEM in the examples of the present invention. Statistic comparison was performed by ANOVA followed by Bonferroni/Dunn test. p<0.05 is considered to be statistically significant.

### [Example 1] Plasmids

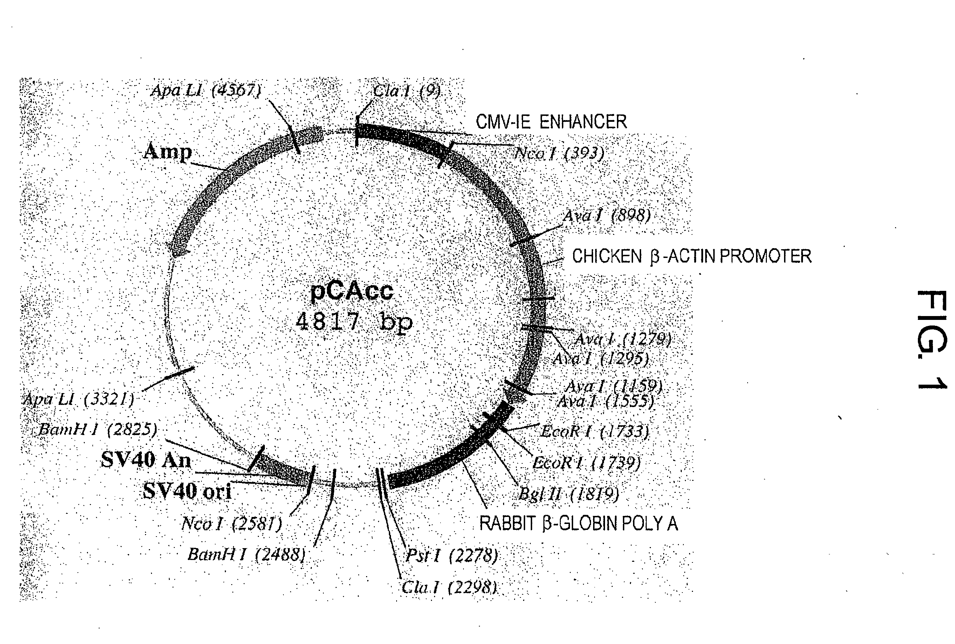

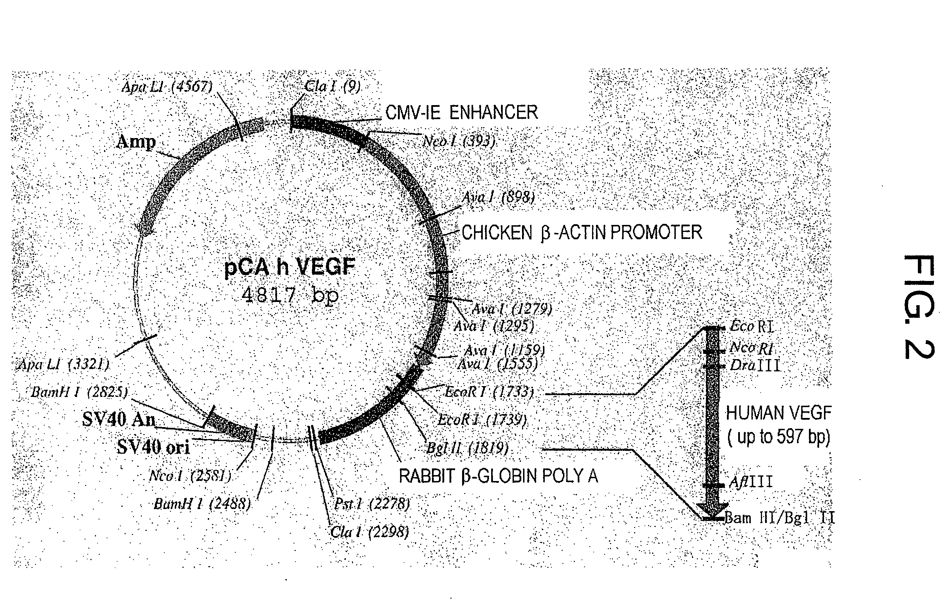

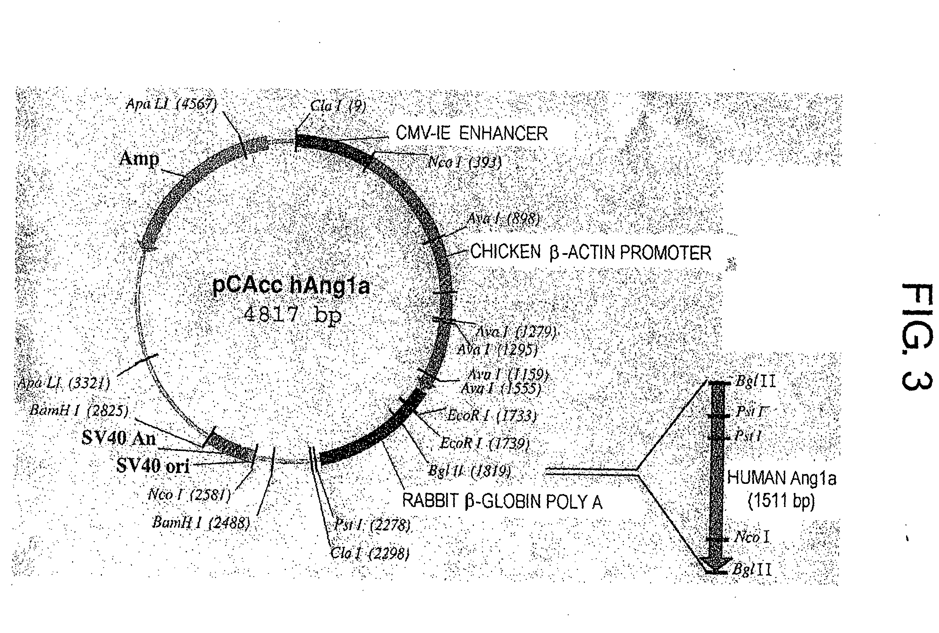

(1) A DNA encoding VEGF was obtained by RT-PCR using RNA extracted from human glioma U251 cells as a template and a set of primers: #1191: (CCGGAATTCACCATGAACTTTCTGCTGTCT/SEQ ID NO: 1) and #1192: (CGCGGATCCTCACCGCCTCGGCTTGTCACA/SEQ ID NO: 2). The amplified fragment was digested with *Eco*RI/*Bam*HI and subcloned into pBluescriptSKII+ at *Eco*RI/*Bam*HI sites. After confirming the nucleotide sequence of the plasmid thus obtained, the plasmid was digested with *Eco*RI/*Bam*HI, and an *Eco*RI/*Bam*HI fragment was subcloned into pCAcc (Fig. 1) at *Eco*RI/*Bgl*II sites to obtain pCAhVEGF (Fig. 2). The control vector pCAcc (Yoshida et al., 1997) was derived from the previously reported pCAGGS (Niwa et al., 1991).
(2) A DNA encoding Angiopoietin 1 (GenBank: HSU83508 (U83508)) was obtained by RT-PCR using RNA extracted from human bone marrow cells (cells obtained by bone marrow aspiration and having ability to remain attached to dishes, so-called bone marrow stroma cells) as a template and a set of primers: #1435: (GAAGATCTATGACAGTTTTCCTTTCCTTTG/SEQ ID NO: 3) and #1436: (GAAGATCTCAAAAATCTAAAGGTCGAATCA/SEQ ID NO: 4). The amplified fragment was digested with *Bgl*II and subcloned into pBluescriptSKII+ at a *Bgl*II site to obtain the plasmid carrying angiopoietin 1. After confirming the nucleotide sequence, the plasmid was digested with *Bgl*II and the isolated *Bgl*II fragment was subcloned into pCAcc (Fig. 1) at the *Bgl*II site to obtain pCAcchAng1a (Fig. 3). The nucleotide sequence of the cloned DNA fragment is shown in SEQ ID NO: 5.

Moreover, PCR was performed using Angiopoietin cDNA fragment of pCAcchAng1a as a template and primer sets #1641: (GGGAATTCACCATGACAGTTTTCCTTTCCTTTGCTTTC/SEQ ID NO: 6) and #1638: (AGCTCCTGGATTATATATGTTTGACG/SEQ ID NO: 7). The amplified fragment was digested with *Eco*RI and used to replace the *Eco*RI fragment of pCAcchAng1a to obtain a plasmid expressing human angiopoietin cDNA, pCAcchAng1, which contained a Kozak consensus sequence. The sequence of the cloned DNA fragment is shown in SEQ ID NO: 8.

### [Example 2] Animal model

New Zealand white rabbits (male, 3.0 to 3.5 kg) were used. After rabbits were anesthetized with a mixture of 3 ml ketaral (Sankyo Co., Ltd.) and 1 ml xylazine (Daiichi Pharmaceutical Co., Ltd.), the femoral artery was exposed by skin incision between the left femoral inguinal ligament and the knee. All of the artery branches were ligated and excised, the femoral artery was removed, and the animals were kept for 10 days to establish a chronic hind limb ischemia model, that is, a rabbit hind limb ischemia model (Takeshita, S., J. Clin. Invest., 1994, 93: 662-670). A comparative assessment was conducted in three groups. Intramuscular injection of plasmids was performed on the 10th day (Day 10) or on the 10th day (Day 10) and 15th day (Day 15) after the establishment of the model (OPE day). Each plasmid (500 µg) was dissolved in 2.5 ml PBS and directly injected into the medial great muscle (2 sites), adductor muscle (2 sites), and semimembranous muscle (1 site) through the incision created in the medial thigh of the ischemic thigh. Each injection was performed slowly using a 27-guage needle at 5 sites, 500 µl each.

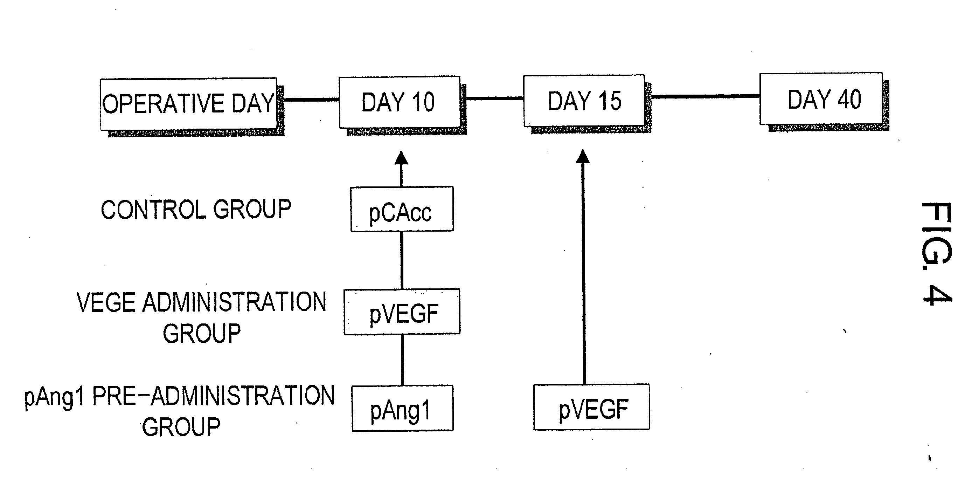

Animal models were divided into six groups as follows according to administration schedule of plasmids (Fig. 4).
1. Control rabbits (Group C) received 500 µg of placebo (pCAcc) on Day 10 (n=8).
2. VEGF alone (Group V) received 500 µg of VEGF on Day 10 (n=10).
3. Ang1 alone (Group A) received 500 µg of Ang1 on Day 10 (n=7).
4. Group A+V received both 500 µg of Ang1 and 500 µg VEGF on Day 10 (n=7).
5. Group A-V received 500 µg of Ang1 on Day 10, followed by administration of 500 µg VEGF on Day 15 (n=10).
6. Group V-A received 500 µg of VEGF,on Day 10, followed by administration of 500 µg of Ang-1 on Day 15 (n=7).

### [Example 3] Assessment of edema

Assessment of edema, the main adverse side effect caused by VEGF, was performed based on measured scrotum size and hind limb circumference. Photographs of the scrotum at the affected site at Day 10, 15, 20, and 40 were taken using a digital camera, and changes were measured by taking the size on Day 10 as 100%. The circumference of the hind limb below the knee was also measured at Day 10, 15, 20, and 40, and the changes in size were evaluated.

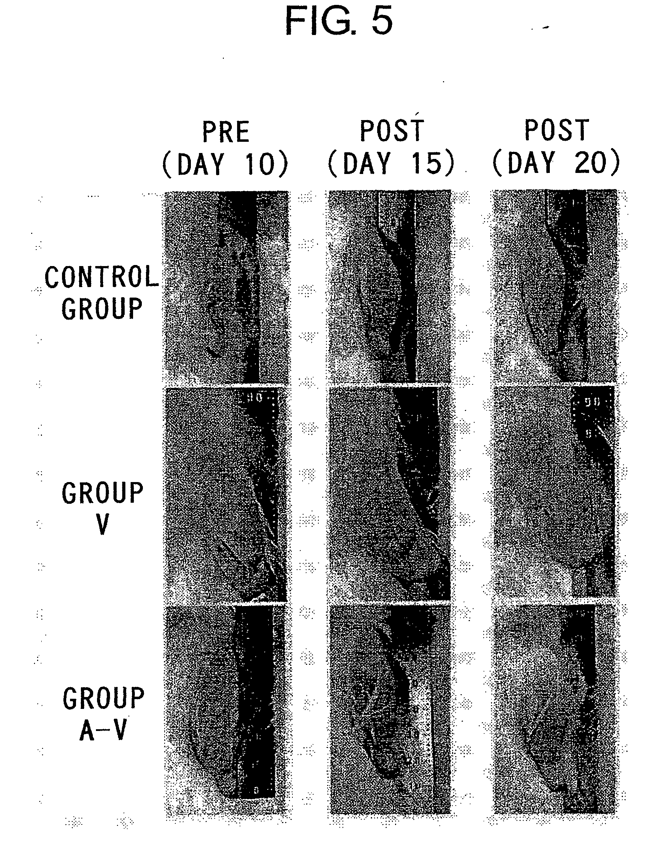

Representative findings of edema by administration of VEGF alone are shown in Fig. 5 and revealed an increased area of the scrotum accompanying venous dilatation. In all groups treated with plasmid DNA, scrotum edema was not followed by inflammatory changes such as necrosis. Average scrotum size before the intramuscular injection of plasmid DNA on Day 10 was 7.14 ± 1.06 cm² and percent change of scrotum size compared to that of Day 10 is shown in Table 1.

**Table 1**

| *Scrotum size (%)* | | | | |
|---|---|---|---|---|
| | *Day 10* | *Day 15* | *Day 20* | *Day 40* |
| Control | 100 | 101.6±2.7 | 101.5 ± 4.1 | 103.3±2.8 |
| Group V | 100 | 140.1±11.1^{b} | 111.2 ± 5.8 | 107.7±1.8 |
| Group A | 100 | 94.6±1.5 | 98.4 ± 3.3 | 98.0±5.5 |
| Group A+V | 100 | 115.5±6.1 | 107.1 ± 4.0 | 97.6±5.1 |
| Group V-A | 100 | 117.9±4.4^{a} | 110.8 ± 7.5 | 118.4±8.6 |
| Group A-V | 100 | 98.4±2.0 | 94.5 ± 3.7 | 94.4±5.1 |

| | | | | |
|---|---|---|---|---|
| ^{a}p<0.05 versus control group | | | | |
| ^{b}p<0.01 versus control group | | | | |

On Day 15, there was a statistically significant increase in edema in Group V (140.1±11.1%, ^{b}p<0.01) and V-A (117.9±4.4%, ^{a}p<0.05), compared with Group C (101.6±2.7%). In Group V and V-A, the size of edema continued to increase on Day 20 and 40, but these increases were of marginal significance. In this experiment, Group A-V did not show any increase of scrotum size throughout study. These results indicate that systemic edema occurred for a short period of 10 days after gene injection in Group V and V-A. In contrast, pre-administration of angiopoietin-1 appeared to prevent the systemic edema induced by VEGF. In addition, the present inventors evaluated hind limb circumference changes as an indicator of local edema in the lower hind limb. Group A-V showed no increased regional edema, whereas an increase of regional edema was noted in Group V, A+V and V-A, compared with Group C.

### [Example 4] Angiographic assessment

Internal iliac artery angiography of affected sites stained by alkaline phosphatase was performed at Day 10 and 40, and an angiographic score (AG score) was used to assess angiogenesis. After anesthetization, a 2.7 Fr infusion catheter was inserted from the right medial carotid artery to the internal iliac artery selectively, 0.25 mg nitrol was injected, then angiography was conducted using 3 ml of contrast medium (3 ml contrast medium was infused within 5 seconds). When 5 mm² grids were placed over the thigh area at 5 mm intervals, a grid crossed by arteries was counted as 1 and a grid crossed by no arteries was counted as 0.. AG score was defined as the total number of grids crossed by arteries/the total number of grids.

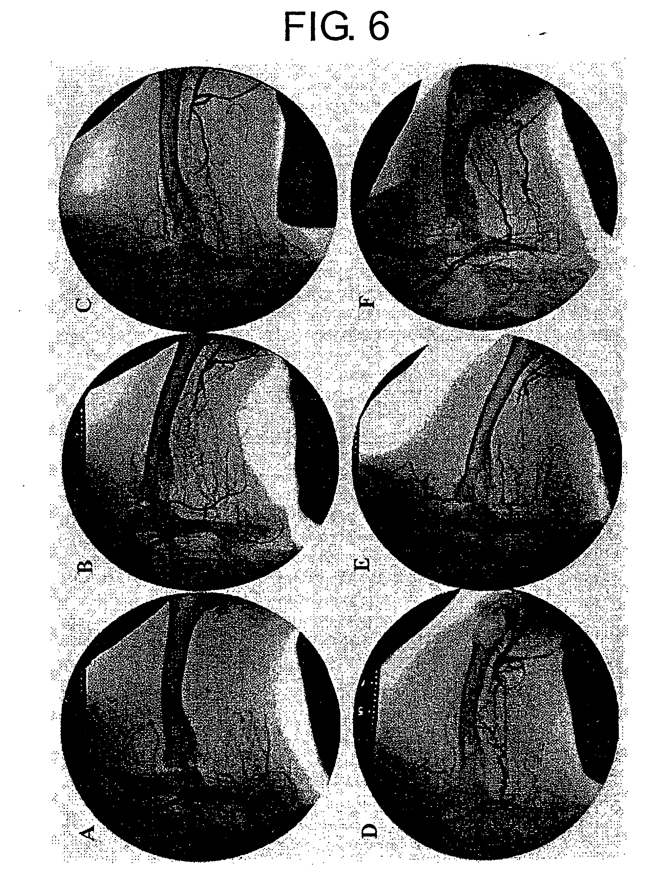

On Day 10, there was no difference in AG scores among Groups C, V, A, A+V, A-V, and V-A (0.08±0.02, 0.11±0.02, 0.07±0.04, 0.08±0.03, 0.07±0.02, and 0.08±0.02, respectively). On Day 40, however, AG scores of Group V, A, and A-V were higher than that of control (Group C: 0.29±0.05, Group V: 0.64±0.05^{a}, Group A: 0.52±0.03^{a}, and Group A-V: 0.58±0.02^{a} ; ^{a}p<0.01) (Table 2). No significant differences were observed among the five test groups. Representative angiographic findings, shown in Fig. 6, indicate the differences in neovascular formation in the medial thigh in terms of number and internal luminal diameter among the six groups. In fact, the internal diameter of the proximal side of the caudal gluteal artery showed that Group A-V had larger vessels than control (Group C: 0.77±0.02 mm and Group A-V: 1.01±0.03 mm^{a}; ^{a}p<0.01) (Table 2). Moreover, Group A-V demonstrated increased vessel cavity (p<0.01) in comparison with Group V (0.76±0.05 mm), suggesting that the pre-administration of angiopoietin-1 contributed to produce larger vessels. There was also a tendency for the formation of more numerous vessels in the Group V and larger internal diameter vessels of the midthigh zone were formed in the Group A-V.

**Table 2**

| | *AG score* | *Arterial diameter (mm)* |
|---|---|---|
| Control | 0.29 ± 0.05 | 0.77 ± 0.02 |
| Group V | 0.64 ± 0.05^{a} | 0.76 ± 0.05 |
| Group A | 0.52 ± 0.03^{a} | 0.85 ± 0.08 |
| Group A+V | 0.49 ± 0.06 | 0.90 ± 0.05 |
| Group V-A | 0.50 ± 0.07 | 0.90 ± 0.04 |
| Group A-V | 0.58 ± 0.02^{a} | 1.01 ± 0.03^{a, b} |

| | | |
|---|---|---|
| AG score = Angiographic score ^{a}p<0.01 versus control group | | |
| ^{b}p<0.01 versus Group V | | |

### [Example 5] Determination of blood pressure ratio

Measurement was taken on Day 10 and Day 40. Doppler was used to find posterior carotid arteries and measure the blood pressure. Blood Pressure Ratio (BPR) was calculated by the blood pressure of the ischemic site/the blood pressure of the non-ischemic site and used as an indicator of body circulation.

Improvement of hemodynamic state was assessed by selective angiography, calf blood pressure (CBP) and resting blood flow (RBF) on Day 10 and 40. Specifically, as described previously (Takeshita et al., 1994), calf blood pressure (CBP) was measured in both hind limbs of each model on Day 10 and Day 40 using a Doppler flowmeter (Datascope, Montvale, NJ) and the cuff, which was connected to a pressure manometer. The CBP ratio was defined for each model as the ratio of the ischemic/normal limb systolic CBP.

Before intramuscular injection of plasmid DNA (Day 10), there was no difference in CBP ratio among all groups, showing that severe ischemia occurred in the operative side of the hind limb. On Day 40, however, CBP ratio increased significantly more in Group A-V (86.0±8.1%, ^{a}p<0.01) than in Group C (51.7±6.9%) (Table 3).

Assessment concerning the improvement of blood perfusion in local tissues was also performed. Specifically, resting blood flow (RBF) of the ischemic limb was measured by attaching a percutaneous probe (P-430, LASERFLO BPM², Vasamedics, St. Paul, Minnesota) to the adductor, medial large and semi-membranous muscles on Day 10 and Day 40. RBF of the ischemic limb was defined as the average peak velocity of four points in the above areas, and the RBF ratio was calculated by dividing RBF on Day 40 by RBF on Day 10. The RBF of the operative limb on Day 10 was similar among six groups. The changes in flow reserve on Day 40 are shown in Table 3. The calculated ratio of RBF on Day 40 to that on Day 10 indicated that there was significant improvement in Group A-V (234.8±12.5%, ^{a}p<0.01) compared with Group C (139.6±10.4%), although a greater degree of blood perfusion was not observed in the other groups.

**Table 3**

| | *CBP ratio (%)* | *Regional RBF ratio (%)* |
|---|---|---|
| Control | 51.7 ± 6.9 | 139.6 ± 10.4 |
| Group V | 68.4 ± 5.6 | 187.6 ± 24.5 |
| Group A | 67.6 ± 5.7 | 207.8 ± 20.5 |
| Group A+V | 82.7 ± 8.8 | 193.3 ± 26.6 |
| Group V-A | 72.4 ± 13.8 | 169.0 ± 24.2 |
| Group A-V | 86.0 ± 8.1^{a} | 234.8 ± 12.5^{a} |
| CBP ratio indicates the calf blood pressure ratio and regional RBF ratio indicates regional resting blood flow ratio. ^{a}P <0.01 versus control group | | |

### [Example 6] Tissue blood flow measurements

Measurements were taken on Day 10 and Day 40 using a tissue blood flow meter. Tissue blood flow was measured at the ischemic medial great muscle (2 sites) and adductor muscle (2 sites), and total values were assessed by taking the value on Day 10 as 100% (BPM2). BPR and BPM2 showed significant improvement in the Ang-1 pre-administrated group compared with the control group.

### [Example 7] VEGF protein measurement

The serum concentration of VEGF in the serum was measured with enzyme-linked immunosorbent assay (ELISA) Human VEGF immunoassay kit (Techne Corp., Minneapolis, MN) on Day 0, 10, 15, 20 and 40. The sensitivity of this assay was 9.0 pg/mL. In every group, the levels of VEGF protein were only moderate: at most 10 to 30 pg/ml throughout the experiment on Day 15.

### [Example 8] Histological assessment

On Day 40, two block-samples of adductor muscle were removed from ischemic limbs for histological analysis. One sample was embedded in OCT compound followed by snap-freezing in liquid nitrogen for alkaline phosphatase stain to determine capillary density. Another was embedded in paraffin after immersion-fixation in formalin for 48 hours for smooth muscle cell α-actin stains to determine arteriole density. Multiple frozen sections were cut (10 µm thickness) on a cryostat and stained for alkaline phosphatase using an indoxyl-tetrazolium method to detect capillary endothelial cells as previously described (Ziada et al., 1984). Capillary density was defined as a mean number of capillaries per 1 mm². For immunohistochemistry, an anti α-smooth muscle actin antibody (1A4, Dako Japan Co., Ltd., Tokyo, Japan) was used as a first antibody and detected by HISTFINE SAB-PO Kit (Nichirei Corp., Tokyo, Japan) with a biotinylated anti-mouse serum and peroxidase-conjugated streptavidin. To identify arterioles and differentiate them from capillaries or venules, fifteen different microscopic fields on three different sections were selected and the positive smooth muscle cells were counted in the same manner. Arteriole density was expressed as a mean number of arterioles per field of view.

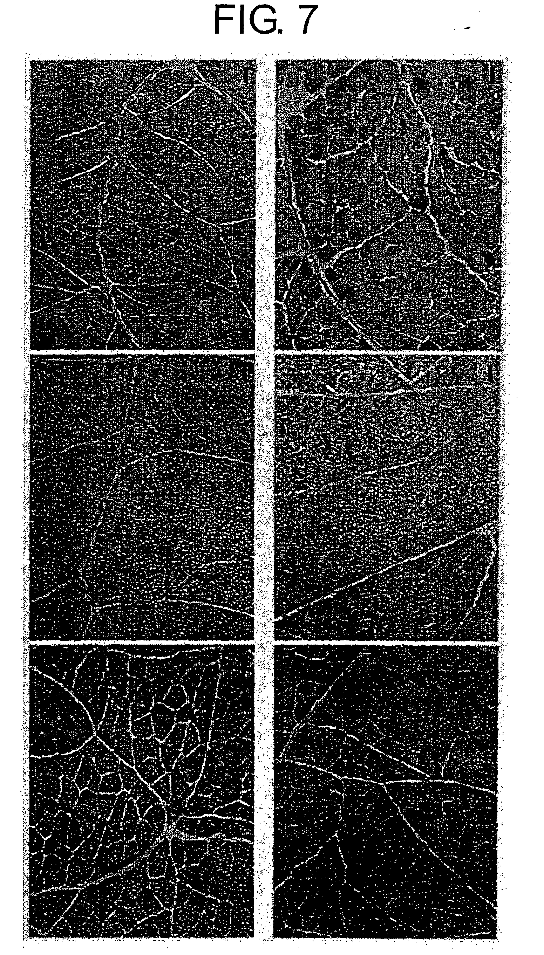

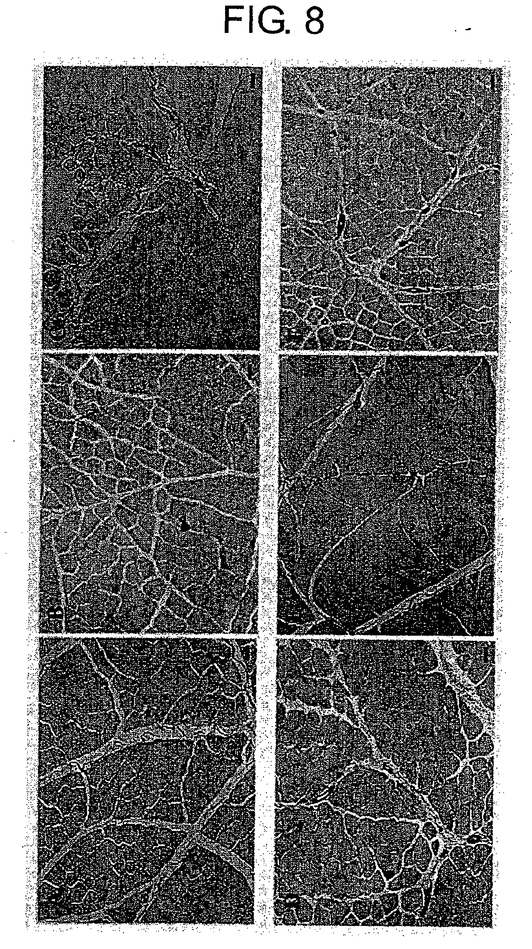

Capillary density (capillary number/mm²) was evaluated as neovascular formation at the capillary (less diameter of 10 µm) level. Density was higher in Group A-V (Group C: 169.9±8.5 and Group A-V: 273.2±25.8^{a}; ^{a}p<0.01 versus Group C) (Table 4). Representative photomicrographs of alkaline-phosphatase stained sections are shown in Fig. 7. Immunohistochemical staining for α-actin in tunica media revealed neovascular formation at the arteriole, which is produced in the late phase of angiogenesis and defined vessels having a diameter range between 10 and 50 µm. Arteriole densities (arteriole number/ field of view) in Group A+V and A-V were significantly higher than that of control (Group C: 4.8±0.5, Group A+V: 10.4±1.7^{a}, Group A-V: 12.4±1.4^{a}; ^{a}p<0.01), but the increases in Group V (6.1±0.8) and A (6.3±1.3) were of marginal significance (Table 4). Moreover, Group A-V showed a greater increase as compared with Group V (^{b}p<0.01) and Group A (^{c}p<0.01). Representative photomicrographs α-actin stained sections are also shown in Fig. 8. These quantitative analyses using alkaline-phosphatase stain and smooth muscle cell α-actin stain indicated that the combination of cytokines, especially pre-administration of angiopoietin-1, produced more functional vascular formation than "solo" administration schedules.

**Table 4**

| | *Capillary density* *(Capillary No.*/*mm*^{*2*}*)* | *Arterial density* *(Arteriole No.*/*field)* |
|---|---|---|
| Control | 169.9 ± 8.5 | 4.8 ± 0.5 |
| Group V | 228.0 ± 3.2 | 6.1 ± 0.8 |
| Group A | 237.2 ± 16.6 | 6.3 ± 1.3 |
| GroupA+V | 242.7 ± 22.8 | 10.4 ± 1.7^{a} |
| Group V-A | 234.6 ± 13.1 | 8.6 ± 0.9 |
| Group A-V | 273.2 ± 25.8^{a} | 12.4 ± 1.4^{a, b, c} |

| | | |
|---|---|---|
| ^{a}p<0.01 versus control group | | |
| ^{b}p<0.01 versus Group V | | |
| ^{c}p<0.01 versus Group A | | |

In therapeutic angiogenesis, production of functional vascular vessels is necessary for the supply of sufficient blood flow in ischemic lesions. However, some studies indicate that VEGF, used widely in clinical studies, is of uncertain value for promotion of functional neovascularization, when VEGF is used alone.

There are two possible reasons for this consideration. First, stenosis or occlusion occurs in a main artery and brings about peripheral ischemia, leading to the expression and activation of the transcription factor, hypoxia-inducible factor-1 (HIF-1). The expression of HIF-1 leads to an increase of the transcription of many angiogenic genes including those encoding nitric oxide synthase and VEGF, and the cascade of angiogenesis progresses (Royen et al., 2001). It is uncertain whether the administration of VEGF gene product alone accomplishes functional angiogenesis. Recent study states that VEGF may stimulate capillary sprouting, but this response does not translate into a significant improvement in collateral flow (Hershey et al., 2001). Furthermore, a study, that examined the improvement of ischemic lesions in rabbit hind limb ischemia by administration of HIF-1α/VP16, showed more effective improvement when compared to the administration of VEGF gene alone (Vincent et al., 2000). Second, although a number of clinical trials using the VEGF gene to treat ischemic diseases have been evaluated and have reported favorable results, some studies have not shown significant improvement (Rajagopalan et al., 2001). In fact, the examples described above showed that administration of VEGF alone induced an increase of capillary number, but did not improve hemodynamic status, and did not mediate vascular maturation. As a result of these accumulated studies, further understanding of combination growth factor therapy or master switch genes is needed if this treatment is to produce clinically beneficial angiogenesis or arteriogenesis (Blau et al., 2001; Carmeliet., 2000; Simons et al., 2000).

The above examples showed that pre-treatment with Ang-1 was more effective in improving hemodynamic status, vascular maturation in late phase angiogenesis, and vascular protection against plasma leakage than administration of Ang-1 or VEGF alone or other schedules of treatment. The data suggest that stimulation by pre-administration of Ang1 promotes angiogenesis in both the early and late phases, contributing to vascular maturation, which may be responsible for improvement of hemodynamic status and vascular protection.

There are two possible mechanisms by which pre-administration of Ang1 might contribute to the promotion of angiogenesis in the early and late phases in this model. First, circulating endothelial precursor cells (CEPs) caused by exogenous Ang1 gene may augment VEGF-induced angiogenesis. CEPs contribute to postnatal neovascularization (Asahara et al., 1999) and one recent study reports that the administration of the Ang1 gene prolongs the mobilization of CEPs compared with the administration of VEGF (Hattori et al., 2001). Overexpression of VEGF165 by adenovirus vector caused mobilization of CEPs to the peripheral blood in mice, peaking at day 2 and returning to control levels by day 14. In contrast, overexpression of Ang1 resulted in increased levels of CEPs, peaking from day 7 to 14 and returning, to control levels by day 28. Accordingly, the more effective angiogenesis produced by combination therapy of VEGF gene and Ang1 gene may occur in the pre-administration of Ang1 because each peaks of CEPs migration caused by administration of these two genes happen at the same phase. In addition, Yamashita et al. demonstrated that ECs and mural cells (pericytes and vascular smooth muscle cells) could be derived from common progenitor cells (Yamashita et al., 2000). They concluded that VEGF is necessary for the ECs differentiation, but differentiation of mural cells could occur independently of exogenous growth factors (Yamashita et al., 2000). Thus, pre-administration of Ang1 gene in the absence of exogenous VEGF until Day 15 could promote migration of circulating precursor cells (CPs) which are composed of CEPs and circulating mural precursor cells (CMPs). In the pre-administration of Ang1 schedule, the ratio of CMPs in CPs population might be higher compared with those by other administration schedules because the phase of VEGF administration (which increased CEPs) is later than other combinations, resulting in production of more mature vessels. Second, pre-administration of the Ang1 gene followed by the VEGF gene may stimulate clear gene switching of Ang2, an important proangiogenic factor, and augment neovascular formation. The expression of Ang2 is necessary for adult angiogenesis and this factor plays an early role at the sites of neovascularization (Malsonpierre et al., 1997). Mandriota et al. also showed that Ang2 mRNA levels were increased by administration of VEGF and decreased by that of Ang1 (Mandriota et al., 1998). Taken together, these findings suggest that in the pre-administration of Ang1 gene schedule, clearer angiogenic switching to endogenous Ang2 could occur and expression of Ang2 could promote angiogenesis longer, compared to the other combination schedules. In the co-administration schedule, the expression of Ang2 would be less than the pre-administration schedules because VEGF and Ang1 have opposite effects on this gene expression. In the post-administration schedule, the period of expression of Ang2 would be shorter than in pre-administration, because of the administration of Ang1 at Day 15.

The present inventors evaluated VEGF-induced edema using scrotum size. The change ratio of scrotum size was sensitive for the evaluation of edema because the scrotum in rabbits is isolated from the body, does not experience strong tissue support, and has abundant areas of connective tissue spaces, which facilitate the accumulation of extravasated fluid. The examples described above showed that only the pre-administration schedule prevented edema. Ang1 may promote endothelial cell firm-attachment to surrounding matrix and cells, resulting in prevention of vascular permeability caused by various stimuli (Thurston et al., 1999). The molecular mechanisms by which Ang1 induces attachment remain to be elucidated, but a recent study demonstrated that Ang1 could directly support cell adhesion mediated by integrins (Carlson et al., 2001). Thurston et al. reported that the resistance to vascular leakage occurred within 48 hours in gene transfer of Ang1 to rats by an adenovirus vector and was verified 7 days later with injection of VEGF protein in same model (Thurston et al., 2000). Actually, the examples revealed that the pre-administration schedule blocked vascular permeability induced by VEGF. In contrast, edema occurred in other treatment schedules, because the resistance to permeability induced by Ang1 did not have time to develop and prevent the vascular permeability caused by VEGF.

In summary, only the pre-administration schedule of Ang1 gene in three combination schedules of VEGF and Ang1 showed effective improvement in ischemic lesions. In the examples, pre-administration of Ang1 gene contributed to significant increased blood pressure in the ischemic limb, improvement of blood flow in ischemic lesions, formation of mature vessels, and prevention of edema. Therefore, priming by Ang-1 gene administration may be beneficial for therapeutic angiogenesis in VEGF gene therapy.

### Industrial Applicability

The present invention provides a method of administering angiopoietin prior to VEGF to obtain a high angiogenic induction effect without the adverse effects of edema. Therefore, the present invention enables gene therapy that prevents the increase in vascular permeability caused by administration of VEGF alone.

## Claims

1. A method of treatment by local administration, said method comprising administering an angiopoietin or a vector encoding an angiopoietin prior to administration of a vector encoding an angiogenic gene or a protein encoded by an angiogenic gene.

2. The method of claim 1, wherein said local administration is intramuscular administration.

3. The method of claim 1 or 2, wherein said angiogenic gene is VEGF121 or VEGF165.

4. The method of any one of claims 1 to 3, wherein the angiopoietin is angiopoietin 1.


SEQUENCE LISTING

<110> DNAVEC RESEARCH INC.

<120> METHOD FOR TREATMENT OF VASCULAR REGENERATION

<130> 65382

<150> PCT/JP 02/05 485
<151> 2002-06-04

<150> JP 2001-174919
<151> 2001-06-08

<160> 8

<170> PatentIn Ver. 2.1

<210> 1
<211> 30
<212> DNA
<213> Artificial Sequence

<220>
<223> Description of Artificial Sequence: an artificially
      synthesized primer sequence

<400> 1
ccggaattca ccatgaactt tctgctgtct                                   30


<210> 2
<211> 30
<212> DNA
<213> Artificial Sequence

<220>
<223> Description of Artificial Sequence: an artificially
      synthesized primer sequence

<400> 2
cgcggatcct caccgcctcg gcttgtcaca                                   30


<210> 3
<211> 30
<212> DNA
<213> Artificial Sequence

<220>
<223> Description of Artificial Sequence: an artificially
      synthesized primer sequence

<400> 3
gaagatctat gacagttttc ctttcctttg                                   30


<210> 4
<211> 30
<212> DNA
<213> Artificial Sequence

<220>
<223> Description of Artificial Sequence: an artificially
      synthesized primer sequence

<400> 4
gaagatctca aaaatctaaa ggtcgaatca                                        30


<210> 5
<211> 1508
<212> DNA
<213> Homo sapiens

<400> 5
agatctatga cagttttcct ttcctttgct ttcctcgctg ccattctgac tcacataggg 60
tgcagcaatc agcgccgaag tccagaaaac agtgggagaa gatataaccg gattcaacat 120
gggcaatgtg cctacacttt cattcttcca gaacacgatg gcaactgtcg tgagagtacg 180
acagaccagt acaacacaaa cgctctgcag agagatgctc cacacgtgga accggatttc 240
tcttcccaga aacttcaaca tctggaacat gtgatggaaa attatactca gtggctgcaa 300
aaacttgaga attacattgt ggaaaacatg aagtcggaga tggcccagat acagcagaat 360
gcagttcaga accacacggc taccatgctg gagataggaa ccagcctcct ctctcagact 420
gcagagcaga ccagaaagct gacagatgtt gagacccagg tactaaatca aacttctcga 480
cttgagatac agctgctgga gaattcatta tccacctaca agctagagaa gcaacttctt 540
caacagacaa atgaaatctt gaagatccat gaaaaaaaca gtttattaga acataaaatc 600
ttagaaatgg aaggaaaaca caaggaagag ttggacacct taaaggaaga gaaagagaac 660
cttcaaggct tggttactcg tcaaacatat ataatccagg agctggaaaa gcaattaaac 720
agagctacca ccaacaacag tgtccttcag aagcagcaac tggagctgat ggacacagtc 780
cacaaccttg tcaatctttg cactaaagaa ggtgttttac taaagggagg aaaaagagag 840
gaagagaaac catttagaga ctgtgcagat gtatatcaag ctggttttaa taaaagtgga 900
atctacacta tttatattaa taatatgccg gaacccaaaa aggtgttttg caatatggat 960
gtcaatgggg gaggttggac tgtaatacaa catcgtgaag atggaagtct agatttccaa 1020
agaggctgga aggaatataa aatgggtttt ggaaatccct ccggtgaata ttggctgggg 1080
aatgagttta tttttgccat taccagtcag aggcagtaca tgctaagaat tgagttaatg 1140
gactgggaag ggaaccgagc ctattcacag tatgacagat tccacatagg aaatgaaaag 1200
caaaactata ggttgtattt aaaaggtcac actgggacag caggaaaaca gagcagcctg 1260
atcttacacg gtgctgattt cagcactaaa gatgctgata atgacaactg tatgtgcaaa 1320
tgtgccctca tgttaacagg aggatggtgg tttgatgctt gtggcccctc caatctaaat 1380
ggaatgttct atactgcggg acaaaaccat ggaaaactga atgggataaa gtggcactac 1440
ttcaaagggc ccagttactc cttacgttcc acaactatga tgattcgacc tttagatttt 1500
tgagatct                                                          1508


<210> 6
<211> 38
<212> DNA
<213> Artificial Sequence

<220>
<223> Description of Artificial Sequence: an artificially
      synthesized primer sequence

<400> 6
gggaattcac catgacagtt ttcctttcct ttgctttc                               38


<210> 7
<211> 26
<212> DNA
<213> Artificial Sequence

<220>
<223> Description of Artificial Sequence: an artificially
      synthesized primer sequence

<400> 7
agctcctgga ttatatatgt ttgacg                                          26


<210> 8
<211> 1511
<212> DNA
<213> Homo sapiens

<400> 8
   gaattcacca tgacagtttt cctttccttt gctttcctcg ctgccattct gactcacata 60
   gggtgcagca atcagcgccg aagtccagaa aacagtggga gaagatataa ccggattcaa 120
   catgggcaat gtgcctacac tttcattctt ccagaacacg atggcaactg tcgtgagagt 180
   acgacagacc agtacaacac aaacgctctg cagagagatg ctccacacgt ggaaccggat 240
   ttctcttccc agaaacttca acatctggaa catgtgatgg aaaattatac tcagtggctg 300
   caaaaacttg agaattacat tgtggaaaac atgaagtcgg agatggccca gatacagcag 360
   aatgcagttc agaaccacac ggctaccatg ctggagatag gaaccagcct cctctctcag 420
   actgcagagc agaccagaaa gctgacagat gttgagaccc aggtactaaa tcaaacttct 480
   cgacttgaga tacagctgct ggagaattca ttatccacct acaagctaga gaagcaactt 540
   cttcaacaga caaatgaaat cttgaagatc catgaaaaaa acagtttatt agaacataaa 600
   atcttagaaa tggaaggaaa acacaaggaa gagttggaca ccttaaagga agagaaagag 660
   aaccttcaag gcttggttac tcgtcaaaca tatataatcc aggagctgga aaagcaatta 720
   aacagagcta ccaccaacaa cagtgtcctt cagaagcagc aactggagct gatggacaca 780
   gtccacaacc ttgtcaatct ttgcactaaa gaaggtgttt tactaaaggg aggaaaaaga 840
   gaggaagaga aaccatttag agactgtgca gatgtatatc aagctggttt taataaaagt 900
   ggaatctaca ctatttatat taataatatg ccggaaccca aaaaggtgtt ttgcaatatg 960
   gatgtcaatg ggggaggttg gactgtaata caacatcgtg aagatggaag tctagatttc 1020
   caaagaggct ggaaggaata taaaatgggt tttggaaatc cctccggtga atattggctg 1080
   gggaatgagt ttatttttgc cattaccagt cagaggcagt acatgctaag aattgagtta 1140
   atggactggg aagggaaccg agcctattca cagtatgaca gattccacat aggaaatgaa 1200
   aagcaaaact ataggttgta tttaaaaggt cacactggga cagcaggaaa acagagcagc 1260
   ctgatcttac acggtgctga tttcagcact aaagatgctg ataatgacaa ctgtatgtgc 1320
   aaatgtgccc tcatgttaac aggaggatgg tggtttgatg cttgtggccc ctccaatcta 1380
   aatggaatgt tctatactgc gggacaaaac catggaaaac tgaatgggat aaagtggcac 1440
   tacttcaaag ggcccagtta ctccttacgt tccacaacta tgatgattcg acctttagat 1500
   ttttgagatc t                                                           1511